(19) Europäisches Patentamt
European Patent Office
Office européen des brevets

(11) Veröffentlichungsnummer : **0 417 875 B1**

(12) # EUROPÄISCHE PATENTSCHRIFT

(45) Veröffentlichungstag der Patentschrift : **12.05.93 Patentblatt 93/19**

(51) Int. Cl.$^5$ : **C07D 403/04,** A01N 43/653

(21) Anmeldenummer : **90250232.7**

(22) Anmeldetag : **11.09.90**

(54) **1-Methoxypyrimidinyl-1H-1,2,4-triazol-3-sulfonsäurenitroanilide, Verfahren zu ihrer Herstellung und ihre Verwendung als Mittel mit herbizider Wirkung.**

Die Akte enthält technische Angaben, die nach dem Eingang der Anmeldung eingereicht wurden und die nicht in dieser Patentschrift enthalten sind.

(30) Priorität : **14.09.89 DE 3931060**

(43) Veröffentlichungstag der Anmeldung : **20.03.91 Patentblatt 91/12**

(45) Bekanntmachung des Hinweises auf die Patenterteilung : **12.05.93 Patentblatt 93/19**

(84) Benannte Vertragsstaaten : **AT BE CH DE DK ES FR GB GR IT LI LU NL SE**

(56) Entgegenhaltungen : **EP-A- 0 246 749**

(73) Patentinhaber : **SCHERING AKTIENGESELLSCHAFT Berlin und Bergkamen**
**Müllerstrasse 170/178 Postfach 65 03 11**
**W-1000 Berlin 65 (DE)**

(72) Erfinder : **Tarara, Gerhard, Dr.**
**Sprengelstrasse 36**
**W-1000 Berlin 65 (DE)**
Erfinder : **Krüger, Gabriele, Dr.**
**Bachstrasse 3**
**W-1000 Berlin 21 (DE)**
Erfinder : **Wegner, Peter, Dr.**
**Münchener Strasse 3**
**W-1000 Berlin 28 (DE)**
Erfinder : **Johann, Gerhard, Dr.**
**Hermsdorfer Damm 147**
**W-1000 Berlin 28 (DE)**
Erfinder : **Rees, Richard, Dr.**
**Speerweg 8**
**W-1000 Berlin 28 (DE)**

EP 0 417 875 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Der Erfindung betrifft neue 1-Methoxypyrimidinyl-1H-1,2,4,-triazol-3-sulfonsäurenitroanilide, Verfahren zu ihrer Herstellung und ihre Verwendung als Mittel mit herbidizer Wirkung.

In der EP-Anmeldung 0 246 749 werden herbizid wirksame Triazolsulfonamide der Formel

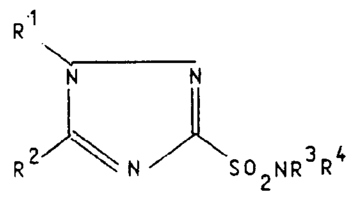

und deren Salze beansprucht, in der

$R^1$ Wasserstoff, ein substituiertes oder unsubstituiertes Alkyl, Alkenyl, Alkinyl, Cycloalkyl, Aryl, Aralkyl, Acyl, Alkoxycarbonyl, Aminocarbonyl oder Sulfonyl oder eine heterocyclische Gruppe,

$R^2$ Wasserstoff, Halogen, Cyano, Hydroxy, Mercapto, ein substituiertes oder unsubstituiertes Alkyl, Alkenyl, Alkinyl, Alkoxy, Alkylthio, Alkylsulfinyl, Alkylsulfonyl, Acyl, Alkoxycarbonyl, Aminocarbonyl oder Amino oder eine heterocyclische Gruppe,

$R^3$ eine substituierte oder unsubstituierte heterocyclische, benzoheterocyclische, Aryl- oder Aralkyl-Gruppe und

$R^4$ Wasserstoff, ein substituiertes oder unsubstituiertes Alkyl, Alkenyl, Alkinyl, Acyl, Alkylsulfonyl, Alkoxycarbonyl, Aminocarbonyl oder Aralkyl oder eine Gruppe der Formel

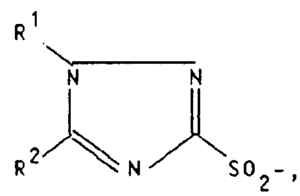

in der $R^1$ und $R^2$ die vorstehend genannten Bedeutungen haben, darstellen.

Wir haben nun gefunden, daß eine spezielle Gruppe von Verbindungen innerhalb dieses breiten Patentanspruches besonders wertvolle Eigenschaften besitzt. Die Auswahl dieser Gruppe war weder aus der früheren Veröffentlichung voraussehbar, noch konnte vorhergesehen werden, daß diese Gruppe von Verbindungen besondere Vorteile zeigen würde.

Gemäß der Erfindung werden bevorzugt 1-Methoxypyrimidinyl-1H-1,2,4-triazol-3-sulfonsäurenitroanilide der allgemeinen Formel I

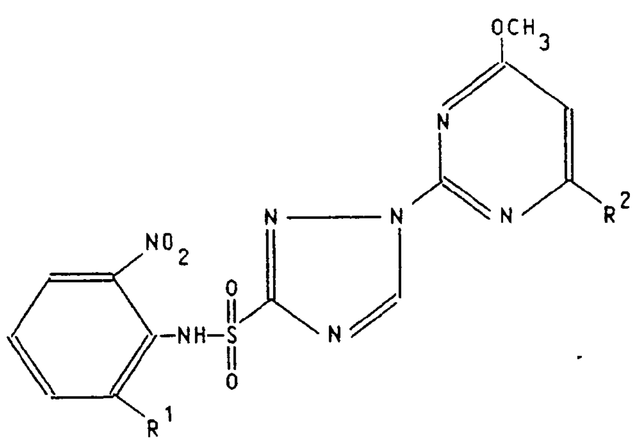

(I) ,

in der

$R^1$ Methyl, Methoxy, Allyloxy oder Propargyloxy und

$R^2$ Methyl oder Methoxy,

bedeuten mit der Einschränkung, daß $R^1$ nicht Methyl ist, wenn $R^2$ Methoxy darstellt.

Die erfindungsgemäßen Verbindungen sind somit 1H-1,2,4-Triazol-3-sulfonsäureamidderivate, die an der Sulfonsäureamidbindung eine Phenylgruppe (mit festgelegten Substituenten) tragen und bei denen das Triazol in 1-Stellung durch eine 4,6-Dimethoxy-pyrimidin-2-yl-gruppe oder eine 4-Methoxy-6-methyl-pyrimidin-2-yl-gruppe substituiert ist.

Die erfindungsgemäßen Verbindungen sind gekennzeichnet durch eine gute herbizide Wirkung, insbesondere durch eine hohe Wirksamkeit gegen wichtige Reisunkräuter verbunden mit einer guten Selektivität in Wasserreis. Diese Kombination einer guten Wirksamkeit gegen wichtige Reisunkräuter mit einer guten Selektivität in Wasserreis ist bei den Verbindungen, die in der EP-Anmeldung 0 246 749 genannt sind, nicht erkennbar.

Die erfindungsgemäßen Verbindungen der allgemeinen Formel I können zum Beispiel hergestellt werden, indem man

A) Nitroanilide der allgemeinen Formel II

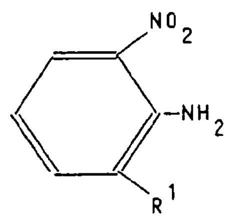

(II),

in der $R^1$ die oben angegebene Bedeutung hat, mit einem Sulfonsäurechlorid der allgemeinen Formel III

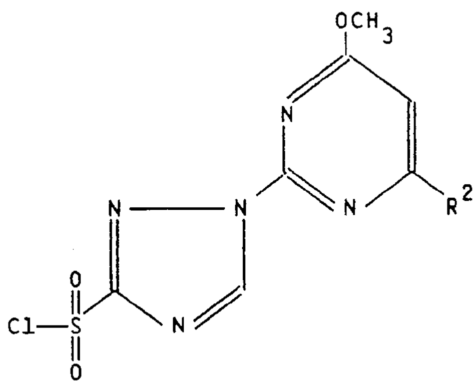

(III) ,

in der $R^2$ die oben angegebene Bedeutung hat, in einem geeigneten Lösungsmittel in Gegenwart eines Säureakzeptors umsetzt, oder

B) eine Verbindung der allgemeinen Formel IV

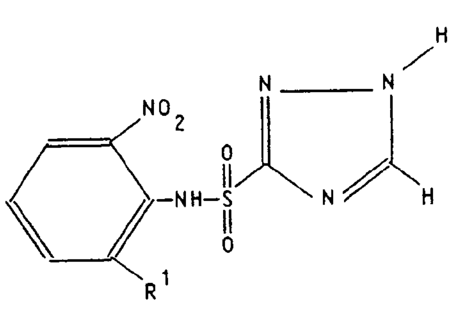

(IV),

in der $R^1$ die oben genannte Bedeutung hat, mit einer Verbindung der allgemeinen Formel V

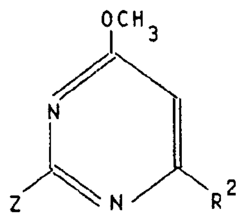

(V) ,

in der Z für Chlor, Brom oder eine Alkyl- oder Arylsulfonylgruppe steht und $R^2$ die oben genannte Bedeutung hat, in einem geeigneten Lösungsmittel in Gegenwart eines säurebindenden Mittels umsetzt.

Die einzelnen Verfahrensvarianten werden vorzugsweise in Gegenwart eines Verdünnungsmittels durchgeführt. Zu diesem Zweck können sämtliche gegenüber den verwendeten Reagenzien inerte Lösungsmittel verwendet werden.

Beispiele für solche Lösungsmittel beziehungsweise Verdünnungsmittel sind Wasser, aliphatische, alicyclische und aromatische Kohlenwasserstoffe, die jeweils gegebenenfalls chloriert sein können, wie zum Beispiel Hexan, Cyclohexan, Petrolether, Ligroin, Benzol, Toluol, Xylol, Methylenchlorid, Chloroform, Kohlenstofftetrachlorid, Ethylenchlorid und Trichlorethylen, Ether, wie zum Beispiel Diisopropylether, Dibutylether, Propylenoxid, Dioxan und Tetrahydrofuran, Ketone, wie zum Beispiel Aceton, Methylethylketon, Methylisopropylketon und Methylisobutylketon, Nitrile, wie zum Beispiel Acetonitril und Propionitril, Alkohole, wie zum Beispiel Methanol, Ethanol, Isopropanol, Butanol und Ethylenglycol, Ester, wie zum Beispiel Ethylacetat und Amylacetat, Säureamide, wie Beispiel Dimethylformamid und Dimethylacetamid, Sulfone und Sulfoxide, wie Beispiel Dimethylsulfoxid und Sulfolan, und Basen, wie zum Beispiel Pyridin.

Die Reaktionen werden vorzugsweise zwischen Raumtemperatur und dem Siedepunkt des jeweiligen Reaktionsgemisches durchgeführt. Die Reaktionen lassen sich bei dem Druck der Umgebung durchführen, wenngleich sie auch bei erhöhtem beziehungsweise vermindertem Druck durchgeführt werden können.

Die Verfahrensvariante A) wird vorzugsweise in chlorierten Kohlenwasserstoffen, wie Dichlormethan oder Dichlorethan, in Anwesenheit eines Katalysators beziehungsweise Säureakzeptors durchgeführt. Beispiele hierfür sind tertiäre Amine, wie zum Beispiel Triethylamin, Diisopropylethylamin, N-Methylmorpholin, 4-Dimethylaminopyridin und Pyridin. Pyridin kann sowohl als Katalysator als auch als Lösungsmittel bei dieser Reaktion verwendet werden.

Die Verfahrensvariante B) wird bevorzugt in inerten Lösungsmitteln, wie Dioxan, Tetrahydrofuran, Dimethylformamid oder N-Methylpyrrolidinon, gegebenenfalls in Anwesenheit eines Katalysators durchgeführt. Beispiele für säurebindende Mittel sind Metallhydride, tert. Amine, wie zum Beispiel Triethylamin oder Diisopropylethylamin und anorganische Basen, wie zum Beispiel Alkali- oder Erdalkalihydroxide oder- carbonate.

Die nach den oben genannten Verfahren hergestellten erfindungsgemäßen Verbindungen können nach den üblichen Verfahren aus dem Reaktionsgemisch isoliert werden, beispielsweise durch Abdestillieren des eingesetzten Lösungsmittels bei normalem oder vermindertem Druck, durch Ausfällen mit Wasser oder durch Extraktion.

Ein erhöhter Reinheitsgrad kann in der Regel durch säulenchromatographische Aufreinigung sowie durch fraktionierte Destillation oder Kristallisation erhalten werden.

Die erfindungsgemäßen Verbindungen stellen in der Regel farb- und geruchlose Kristalle dar, die wenig löslich in Wasser und in aliphatischen Kohlenwasserstoffen, wie Petrolether, Hexan, Pentan und Cyclohexan, gut löslich in halogenierten Kohlenwasserstoffen, wie Chloroform, Methylenchlorid und Tetrachlorkohlenstoff, aromatischen Kohlenwasserstoffen, wie Benzol, Toluol und Xylol, Ethern, wie Diethylether, Tetrahydrofuran und Dioxan, Carbonsäurenitrilen, wie Acetonitril, Alkoholen, wie Methanol und Ethanol, Carbonsäureamiden, wie Dimethylformamid, und Sulfoxiden, wie Dimethylsulfoxid, sind.

Die Verbindungen der allgemeinen Formeln II, III, IV und V können nach in der Literatur beschriebenen Verfahren hergestellt werden, insbesondere nach den in der EP-Anmeldung 0 246 749 genannten Verfahren.

Wie bereits weiter oben ausgeführt, zeigen die erfindungsgemäßen Verbindungen eine gute Herbizidwirkung. Diese Wirkung erstreckt sich auf monokotyle und dikotyle Unkräuter bei einer guten Selektivität in verschiedenen Nutzpflanzen, wie zum Beispiel in Wasserreis.

Die erfindungsgemäßen Verbindungen können zum Beispiel bei den folgenden Pflanzengattungen verwendet werden:

Dikotyle Unkräuter der Gattungen Sinapis, Lepidium, Galium, Stellaria, Matricaria, Anthemis, Galinsoga, Chenopodium, Brassica, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Lamium, Veronica, Abutilon, Datura, Viola, Galeopsis, Papaver, Centaurea und Chrysanthemum.

Monokotyle Unkräuter der Gattungen Avena, Alopecurus, Echinochloa, Setaria, Panicum, Digitaria, Poa, Eleusine, Brachiaria, Lolium, Bromus, Cyperus, Agropyron, Sagittaria, Monochoria, Fimbristylis, Eleocharis, Ischaemum und Apera.

Die Aufwandmengen schwanken je nach Anwendungsart im Vor- und Nachauflauf in Grenzen zwischen 0,01 bis 1 kg/ha.

Die erfindungsgemäßen Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden. Sofern eine Verbreiterung des Wirkungsspektrums beabsichtigt ist, können auch andere Herbizide zugesetzt werden.

Beispielsweise eignen sich als herbizid wirksame Mischungspartner diejenigen Wirkstoffe, die in Weed Abstracts, Vol. 38, No. 3, 1989, unter dem Titel "List of common names and abbreviations employed for currently used herbicides and plant growth regulators in Weed Abstracts" aufgeführt sind.

Eine Förderung der Wirkintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Zweckmäßig werden die gekennzeichneten Wirkstoffe oder deren Mischungen in Form von Zubereitungen, wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier- und/oder Dispergierhilfsmitteln angewandt.

Geeignete flüssige Trägerstoffe sind zum Beispiel aliphatische und aromatische Kohlenwasserstoffe, wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Bentonit, Silicagel, Talkum, Kaolin, Attapulgit, Kalkstein, und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des beziehungsweise der Wirkstoffe(s) in den verschiedenen Zubereitungen kann in weiten Grenzen variieren. Beispielsweise enthalten die Mittel etwa 10 bis 90 Gewichtsprozent Wirkstoff, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen etwa 100 bis 1000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, zum Beispiel durch Mahl- oder Mischverfahren, durchgeführt werden. Gewünschtenfalls können Zubereitungen der Einzelkomponenten auch erst kurz vor ihrer Verwendung gemischt werden, wie es zum Beispiel im sogenannten Tankmixverfahren in der Praxis durchgeführt wird.

Zur Herstellung der verschiedenen Zubereitungen werden zum Beispiel die folgenden Bestandteile eingesetzt:

A) **Spritzpulver**

1.) 25 Gewichtsprozent Wirkstoff
60 Gewichtsprozent Kaolin
10 Gewichtsprozent Kieselsäure
5 Gewichtsprozent einer Mischung aus dem Calciumsalz der Ligninsulfonsäure und dem Natriumsalz des N-Methyl-N-oleyl-taurins

2.) 40 Gewichtsprozent Wirkstoff
25 Gewichtsprozent Tonmineralien
25 Gewichtsprozent Kieselsäure
10 Gewichtsprozent einer Mischung aus dem Calciumsalz der Ligninsulfonsäure und Alkylphenylpolyglycolethern

B) **Paste**

45 Gewichtsprozent Wirkstoff
5 Gewichtsprozent Natriumaluminiumsilikat
15 Gewichtsprozent Cetylpolyglycolether mit 8 Mol Ethylenoxid
2 Gewichtsprozent Spindelöl
10 Gewichtsprozent Polyethylenglycol

23 Gewichtsprozent Wasser

C) **Emulsionskonzentrat**

25 Gewichtsprozent Wirkstoff

15 Gewichtsprozent Cyclohexanon

55 Gewichtsprozent Xylol

5 Gewichtsprozent einer Mischung aus dem Calciumsalz der Dodecylbenzolsulfonsäure und Nonylphenylpolyoxyethylen

Die nachstehenden Beispiele erläutern die Herstellung der erfindungsgemäßen Verbindungen:

## Beispiel 1

1-(4,6-Dimethoxy-pyrimidin-2-yl)-1H-1,2,4-triazol-3-sulfonsäure-(6-nitro-2-propargyloxyanilid)

3,23 g (10 mMol) 1H-1,2,4-Triazol-3-sulfonsäure-6-nitro-2-propargyloxyanilid und 2,76 g (20 mMol) Kaliumcarbonat in 25 ml Dimethylformid werden unter Schutzgas 10 Minuten auf 50°C erhitzt. Nach dem Abkühlen auf 0°C und Zugabe von 2,18 g (10 mMol) 4,6-Dimethoxy-2-methylsulfonylpyrimidin läßt man die Mischung auf Raumtemperatur erwärmen und rührt sie 20 Stunden nach. Die Reaktionslösung wird im 300 ml eiskalte, gesättigte Natriumchlorid-Lösung eingerührt und mit 2 N Salzsäurelösung angesäuert (pH 4). Die Kristalle werden abgesaugt und durch Chromatographie an Kieselgel mit Hexan/Essigestergemischen (0 bis 100 % Essigester) gereinigt.

Ausbeute: 2,77 g = 60 % der Theorie

Fp. : 177 - 181 °C

Analog wurden auch die folgenden erfindungsgemäßen Verbindungen hergestellt:

| Beispiel | Name der Verbindung | Physikalische Konstante |
|---|---|---|
| 2 | 1-(4-Methoxy-6-methyl-pyrimidin-2-yl)-1H-1,2,4-triazol-3-sulfonsäure-(2-methyl-6-nitroanilid) | Fp.: 203-204 °C |
| 3 | 1-(4,6-Dimethoxy-pyrimidin-2-yl)-1H-1,2,4-triazol-3-sulfonsäure-(2-methoxy-6-nitroanilid) | Fp.: 226-229 °C |
| 4 | 1-(4,6-Dimethoxy-pyrimidin-2-yl)-1H-1,2,4-triazol-3-sulfonsäure-(2-allyloxy-6-nitroanilid) | Fp.: 134-137 °C |
| 5 | 1-(4-Methoxy-6-methyl-pyrimidin-2-yl)-1H-1,2,4-triazol-3-sulfonsäure-(2-methoxy-6-nitroanilid) | Fp.: 193-196 °C |
| 6 | 1-(4-Methoxy-6-methyl-pyrimidin-2-yl)-1H-1,2,4-triazol-3-sulfonsäure-(2-allyloxy-6-nitroanilid) | Fp.: 179-184 °C |
| 7 | 1-(4-Methoxy-6-methyl-pyrimidin-2-yl)-1H-1,2,4-triazol-3-sulfonsäure-(6-nitro-2-propargyloxyanilid) | Fp.: 187-191 °C |

Die folgenden Beispiele erläutern die Anwendungsmöglichkeiten der erfindungsgemäßen Verbindungen:

**Beispiel A**

Im Gewächshaus wurden die in der Tabelle aufgeführten Verbindungen mit den ebenfalls erwähnten Aufwandmengen appliziert. Hierzu wurden die Wirkstoffe in Form von Zubereitungen auf die Wasseroberfläche in Gefäße mit 1500 ml Wasser pipettiert. Die Testpflanzenarten wurden im Vorauflauf und im 1- bis 5-Blatt-Stadium eingesetzt. Drei Wochen nach der Applikation wurde die Schädigung der Pflanzen bonitiert. Die erfindungsgemäßen Verbindungen zeigten eine starke Wirkung gegen Cyperus difformis und Eleocharis acicularis bei gleichzeitiger Selektivität in Wasserreis.

In der folgenden Tabelle bedeuten:

0 = keine Schädigung
1 = schwache Schädigung
2 = mittlere Schädigung
3 = starke Schädigung
4 = total vernichtet

ORYSA = Oryza sativa
CYPDI = Cyperus difformis
ELOAC = Eleocharis acicularis

| Erfindungsgemäße Verbindungen | Wasserapplikation kg Wirkstoff/ha | ORYSA | CYPDI | ELOAC |
|---|---|---|---|---|
| Beispiel 1 | 0,1 | 0 | 4 | 4 |
| Beispiel 2 | 0,1 | 0 | 4 | 4 |
| Beispiel 3 | 0,1 | 0 | 4 | 4 |
| Beispiel 4 | 0,1 | 0 | 4 | 4 |
| Beispiel 6 | 0,1 | 0 | 4 | 4 |
| Beispiel 7 | 0,1 | 0 | 4 | 4 |
| Unbehandelt | - | 0 | 0 | 0 |

**Beispiel B**

Im Gewächshaus wurden die in der Tabelle aufgeführten Verbindungen mit den ebenfalls erwähnten Aufwandmengen appliziert. Hierzu wurden die Wirkstoffe in Form von Zubereitungen auf die Wasseroberfläche in Gefäße mit 1500 ml Wasser pipettiert. Die Testpflanzenarten wurden im Vorauflauf und im 1- bis 5-Blatt-Stadium eingesetzt. Drei Wochen nach der Applikation wurde die Schädigung der Pflanzen bonitiert. Die erfindungsgemäße Verbindung zeigte eine starke Wirkung gegen Monochoria vaginalis bei gleichzeitiger Selektivität in Wasserreis. Das Vergleichsmittel war schwächer wirksam.

In der folgenden Tabelle bedeuten:

0 = keine Schädigung
1 = schwache Schädigung
2 = mittlere Schädigung
3 = starke Schädigung
4 = total vernichtet

ORYSA: Oryza sativa
MOOVA: Monochoria vaginalis

| Erfindungsgemäße Verbindung | Wasserapplikation kg Wirkstoff/ha | ORYSA | MOOVA |
|---|---|---|---|
| Beispiel 3 | 0,05 | 0 | 4 |
| Unbehandelt | - | 0 | 0 |
| Vergleichsmittel | | | |
| Bensulfuron-methyl | 0,05 | 0 | 3 |

**Beispiel C**

Im Gewächshaus wurden die in der Tabelle aufgeführten Verbindungen mit den ebenfalls erwähnten Aufwandmengen appliziert. Hierzu wurden die Wirkstoffe in Form von Zubereitungen auf die Wasseroberfläche in Gefäße mit 1500 ml Wasser pipettiert. Die Testpflanzenarten wurden im Vorauflauf und im 1- bis 5-Blatt-Stadium eingesetzt. Drei Wochen nach der Applikation wurde die Schädigung der Pflanzen bonitiert. Die erfindungsgemäße Verbindung zeigte eine starke Wirkung gegen Scirpus juncoides bei gleichzeitiger Selektivität in Wasserreis. Das Vergleichsmittel war schwächer wirksam.

In der folgenden Tabelle bedeuten:

0 = keine Schädigung
1 = schwache Schädigung
2 = mittlere Schädigung
3 = starke Schädigung
4 = total vernichtet

ORYSA = Oryza sativa
SCPJU = Scirpus juncoides

| Erfindungsgemäße Verbindung | Wasserapplikation kg Wirkstoff/ha | ORYSA | SCPJU |
|---|---|---|---|
| Beispiel 4 | 0,05 | 0 | 4 |
| Unbehandelt | - | 0 | 0 |
| Vergleichsmittel | | | |
| Bensulfuron-methyl | 0,05 | 0 | 3 |

**Beispiel D**

Im Gewächshaus wurden die in der Tabelle aufgeführten Verbindungen mit den ebenfalls erwähnten Aufwandmengen appliziert. Hierzu wurden die Wirkstoffe in Form von Zubereitungen auf die Wasseroberfläche in Gefäße mit 1500 ml Wasser pipettiert. Die Testpflanzenarten wurden im Vorauflauf und im 1- bis 5-Blatt-Stadium eingesetzt. Drei Wochen nach der Applikation wurde die Schädigung der Pflanzen bonitiert. Die erfindungsgemäße Verbindung zeigte eine starke Wirkung gegen Cyperus serotinus bei gleichzeitiger Selektivität in Wasserreis. Das Vergleichsmittel war schwächer wirksam.

In der folgenden Tabelle bedeuten:

0 = keine Schädigung
1 = schwache Schädigung
2 = mittlere Schädigung
3 = starke Schädigung
4 = total vernichtet

ORYSA = Oryza sativa
CYPSE = Cyperus serotinus

| Erfindungsgemäße Verbindung | Wasserapplikation kg Wirkstoff/ha | ORYSA | CYPSE |
|---|---|---|---|
| Beispiel 1 | 0,05 | 0 | 4 |
| Unbehandelt | - | 0 | 0 |
| Vergleichsmittel | | | |
| Bensulfuron-methyl | 0,05 | 0 | 0 |

## Patentansprüche

1. 1-Methoxypyrimidinyl-1H-1,2,4-triazol-3-sulfonsäurenitroanilide der allgemeinen Formel I

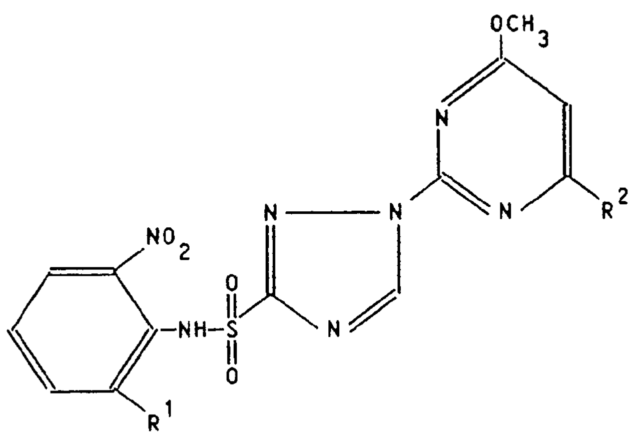

(I),

in der
$R^1$ Methyl, Methoxy, Allyloxy oder Propargyloxy und
$R^2$ Methyl oder Methoxy
bedeuten mit der Einschränkung, daß $R^1$ nicht Methyl ist, wenn $R^2$ Methoxy darstellt.

2. Verfahren zur Herstellung von Verbindungen der allgemeinen Formel I gemäß Anspruch 1, dadurch gekennzeichnet, daß man
A) Nitroaniline der allgemeinen Formel II

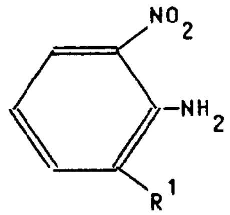

(II),

in der $R^1$ die oben angegebene Bedeutung hat, mit einem Sulfonsäurechlorid der allgemeinen Formel III

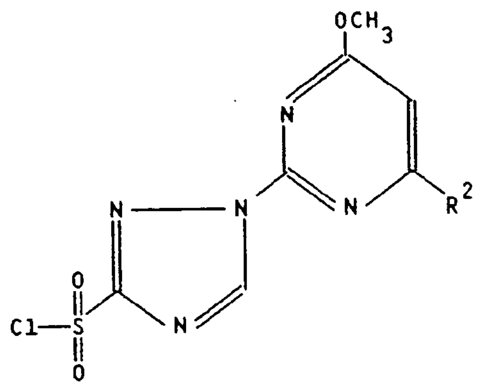

(III) ,

in der $R^2$ die oben angegebene Bedeutung hat, in einem geeigneten Lösungsmittel in Gegenwart eines Säureakzeptors umsetzt, oder

B) eine Verbindung der allgemeinen Formel IV

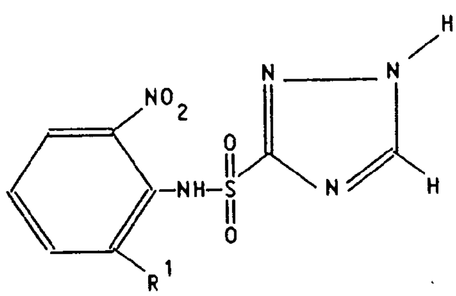

(IV),

in der $R^1$ die oben genannte Bedeutung hat, mit einer Verbindung der allgemeinen Formel V

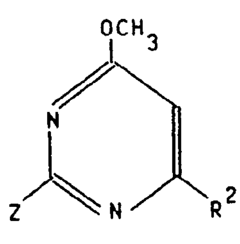

(V) ,

in der Z für Chlor, Brom oder eine Alkyl- oder Arylsulfonylgruppe steht und $R^2$ die oben genannte Bedeutung hat, in einem geeigneten Lösungsmittel in Gegenwart eines säurebindenden Mittels umsetzt.

3. Mittel mit herbizider Wirkung, gekennzeichnet durch einen Gehalt an mindestens einer Verbindung gemäß Anspruch 1.

4. Verwendung von Mitteln gemäß dem Anspruch 3 zur Bekämpfung monokotyler und dikotyler Unkrautarten in Reiskulturen.

5. Verfahren zur Herstellung von Mitteln mit herbizider Wirkung, dadurch gekennzeichnet, daß man Verbindungen der allgemeinen Formel I gemäß Anspruch 1 mit Träger- und/oder Hilfsstoffen vermischt.

## Claims

1. 1-(Methoxypyrimidinyl)-1H-1,2,4-triazole-3-sulphonamide nitroanilides of general formula I

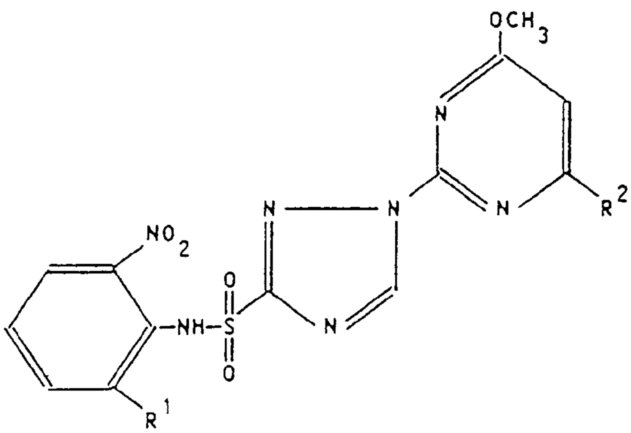

(I)

in which
$R^1$ is methyl, methoxy, allyloxy or propargyloxy, and
$R^2$ is methyl or methoxy,
with the proviso that $R^1$ is not methyl, if $R^2$ is methoxy.

2. Process for the preparation of compounds of general formula I, according to claim 1, characterised by
A) reacting nitroanilines of general formula II

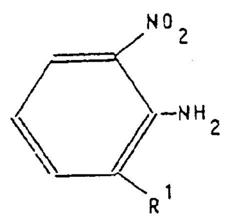

(II)

where $R^1$ has the meaning given above, with a sulphonyl chloride of general formula III

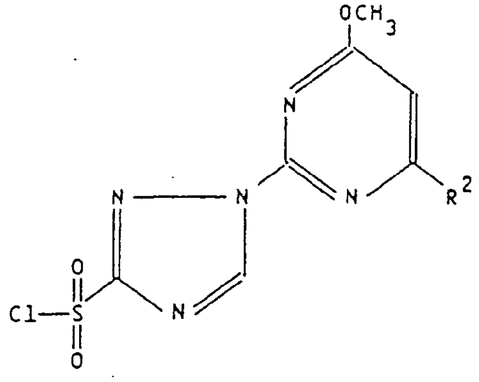

(III)

where $R^2$ has the meaning given above, in a suitable solvent in the presence of an acid acceptor, or

B) reacting a compound of general formula IV

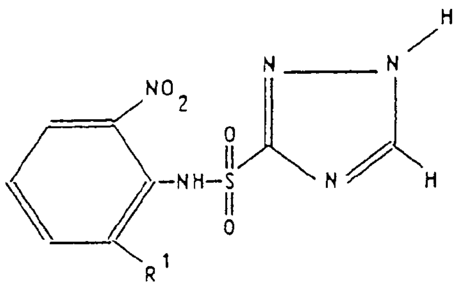

(IV)

where $R^1$ has the meaning given above, with a compound of general formula V

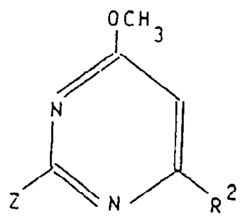

(V)

where Z is chlorine, bromine or an alkyl- or arylsulphonyl group and $R^2$ has the meaning given above, in a suitable solvent in the presence of an acid binding agent.

3. Herbicidal composition characterised by a content of at least one compound according to claim 1.

4. Use of compositions according to claim 3 for combating monocotyledonous and dicotyledonous weeds in rice cultures.

5. Process for the preparation of compositions, having herbicidal activity, characterised by mixing compounds of general formula I, according to claim 1, with carriers and/or diluents.

## Revendications

1. Nitroanilides d'acides 1-méthoxypyrimidinyl-1H-1,2,4-triazole-3-sulfoniques de formule générale I ci-dessous

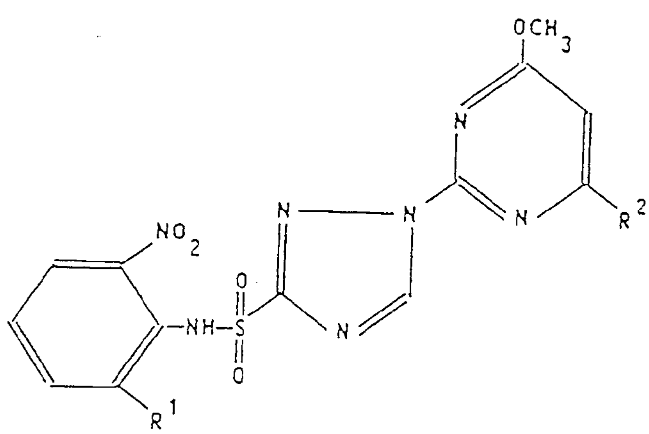

(I),

dans laquelle

$R^1$ désigne un groupe méthyle, méthoxy, allyloxy ou propargyloxy et

$R^2$ le groupe méthyle ou méthoxy,

avec la condition que $R^1$ ne soit pas le groupe méthyle si $R^2$ est un groupe méthoxy.

**2.** Procédé de préparation des composés de formule I selon la revendication 1, procédé caractérisé en ce que :

A) on fait réagir des nitroanilines de formule II

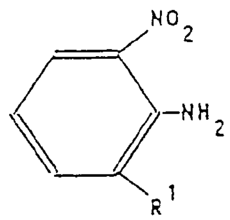

(II),

avec un chlorure d'acide sulfonique de formule III

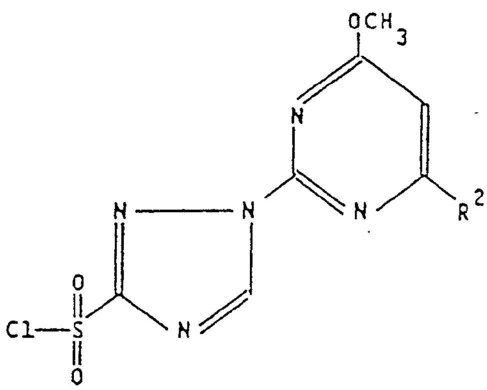

(III) ,

dans un solvant approprié en présence d'un accepteur d'acides, ou bien

B) on fait réagir un composé de formule IV

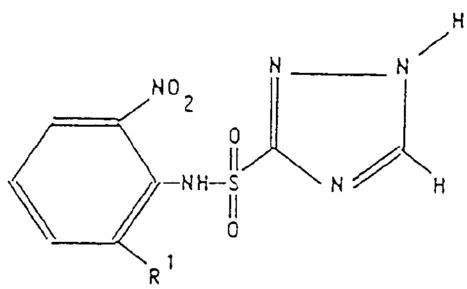

(IV),

avec un composé de formule V

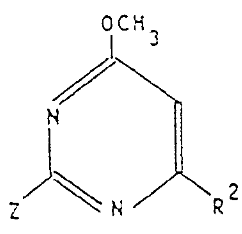

(V) ,

Z désignant le chlore ou le brome ou un groupe alkyl- ou arylsulfonyle, dans un solvant approprié en présence d'un agent fixant les acides.

3. Produits herbicides caractérisés en ce qu'ils contiennent un ou plusieurs composés de la revendication 1.

4. Emploi des produits de la revendication 3 pour lutter contre des mauvaises herbes monocotylédones et dicotylédones dans des cultures de riz.

5. Procédé de préparation de produits herbicides, caractérisé en ce que l'on mélange des composés de formule I selon la revendication 1 avec des matières de support et/ou des adjuvants.